# FASCICULE DE BREVET EUROPEEN

(11) **EP 1 138 346 B1**
(45) Date de publication et mention de la délivrance du brevet: **24.05.2006**
(21) Numéro de dépôt: 01400660.5
(22) Date de dépôt: 14.03.2001
(51) Int. Cl.: A61N 1/368

(54) **Dispositif médical implantable actif comportant des moyens de mesure de bioimpédance transseptale**
Aktive implantierbare medizinische Vorrichtung mit Mitteln zur Messung der transseptalen Bioimpedanz
Active implantable medical device having means for measuring transseptal bioimpedance

(30) Priorité: 14.03.2000 FR 0003243
(43) Date de publication de la demande: 04.10.2001
(73) Titulaire: ELA MEDICAL, 92541 Montrouge (FR)
(72) Inventeur: Dal Molin, Renzo, 92320 Chatillon (FR)
(74) Mandataire: Dupuis-Latour, Dominique

(56) Documents cités:
- WO-A-99/30777

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite" dans lesquelles des électrodes sont placées en une pluralité de sites respectifs distincts comportant au moins un site ventriculaire et un site auriculaire. Il peut s'agir d'une prothèse de type "triple chambre" (stimulation atriale droite et double stimulation ventriculaire) ou "quadruple chambre" (double stimulation atriale et double stimulation ventriculaire).

Le pilotage de la stimulation implique l'ajustement permanent de divers paramètres tels que fréquence de stimulation, délai atrioventriculaire (DAV) ou encore délai interventriculaire dans le cas d'une stimulation biventriculaire.

Ces divers paramètres sont ajustés en fonction de signaux délivrés par des capteurs, par exemple de ventilation-minute (MV), qui est un facteur représentatif des besoins métaboliques instantanés du patient, évalué par mesure de bioimpédance transpulmonaire, c'est à dire entre le coeur et le boîtier du stimulateur, situé dans le haut du thorax.

Un autre facteur qu'il est souhaitable de connaître est le débit cardiaque car il peut être intéressant, tout particulièrement avec un stimulateur multisite, d'obtenir une indication de ce débit et donc de la fraction d'éjection, qui est le paramètre hémodynamique de référence pour optimiser la stimulation sur les différents sites.

Ce débit peut être évalué par mesure de la pression intracardiaque, comme cela est par exemple proposé par le WO-A-99/34863 (Pacesetter AB), mais au prix d'une sonde spécifique incorporant un capteur piézoélectrique et d'une électronique associée particulière pour exploiter les signaux issus de ce capteur, les convertir et les transmettre au microprocesseur du stimulateur.

Un autre paramètre corrélé au débit cardiaque est l'impédance transvalvulaire, généralement mesurée sur le coeur droit, comme cela est par exemple proposé par le US-A-5 154 171 (Chirife). Ce document propose d'effectuer la mesure de bioimpédance transvalvulaire par injection d'une impulsion de courant entre deux sites ventriculaires et recueil d'un potentiel différentiel entre ces deux mêmes points. En pratique, on constate cependant que cette configuration (configuration bipolaire) d'injection/recueil se révèle sensible au mouvement des sondes et ne permet pas une mesure fiable et précise de l'impédance transvalvulaire et par voie de conséquence de la fraction d'éjection.

Le WO-A-9930777 décrit également un dispositif mesurant des valeurs de bioimpédance intracardiaque corrélées à des paramètres hémodynamiques, mais au prix de commutations complexes et multiples entre le circuit de mesure et les diverses électrodes implantées.

L'un des buts de l'invention est de remédier à ces inconvénient en proposant une nouvelle configuration de mesure de l'impédance intracardiaque procurant une image plus fiable et plus précise de la fraction d'éjection, notamment pour piloter le délai interventriculaire (dans le cas d'une stimulation biventriculaire) ou pour piloter la fréquence de stimulation et/ou le délai atrioventriculaire.

Plus précisément, l'invention concerne un dispositif médical du type général connu d'après le US-A-5 154 171 précité, correspondant au préambule de la revendication 1. L'invention propose une configuration d'injection/recueil avec un site commun, telle que spécifiée par la partie caractérisante de la revendication 1 (avec deux variantes alternatives).

Les sous-revendications visent des formes de réalisation préférentielles avantageuses.

On va maintenant décrire de façon plus détaillée la manière de mettre en oeuvre l'invention, en référence aux dessins annexés.
La figure 1 illustre une configuration, selon l'invention, pour la mesure de l'impédance auriculo-ventriculaire transseptale selon l'invention.
La figure 2 illustre une variante de la configuration de la figure 1.

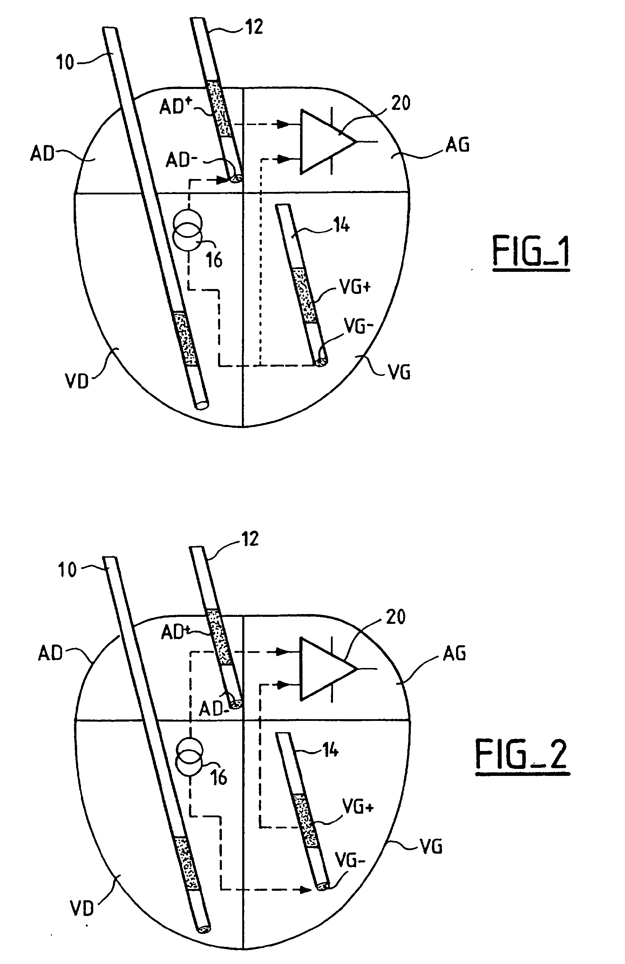

La figure 1 représente schématiquement un muscle cardiaque avec ses quatre cavités : oreillette droite AD, oreillette gauche AG, ventricule droit VD et ventricule gauche VG.

Une sonde ventriculaire 10 est introduite dans le ventricule droit VD (pour mémoire, car cette sonde 10 ne joue pas de rôle pour la mise en oeuvre particulière de la présente invention).

Une sonde auriculaire 12 est introduite dans l'oreillette droite AD, avec une électrode proximale annulaire référencée AD+ et une électrode distale d'extrémité référencée AD-.

Il est également prévu une sonde 14 sur le ventricule gauche VG, avec une électrode proximale annulaire référencée VG+ et une électrode distale d'extrémité référencée VG-.

Les électrodes des sondes sont reliées à un boîtier comprenant divers circuits de détection, de stimulation et de commande, par exemple un boîtier de stimulateur multisite tel que celui décrit dans le EP-A-0 925 806 (ELA Médical) auquel on pourra se référer pour de plus amples détails.

L'invention propose d'effectuer une mesure de bioimpédance transseptale oblique, c'est-à-dire entre l'oreillette droite AD et le ventricule gauche VG.

Cette mesure est réalisée par injection d'une impulsion de courant (schématisée par le générateur de courant 16) entre deux points, et recueil d'un potentiel différentiel (schématisé par l'amplificateur opérationnel 20) entre deux points.

L'injection et le recueil des signaux en ces différents points peuvent être opérés au moyen d'un circuit tel que celui décrit dans le US-A-5 154 171 précité, qui effectue par cette technique une mesure d'impédance transvalvulaire (entre oreillette droite et ventricule droit), ou encore au moyen d'un circuit tel que celui servant à la mesure de la ventilation-minute (MV) sur les dispositifs existants.

Pour réaliser la mesure d'impédance transseptale oblique selon l'invention, les sites d'injection et de recueil sont modifiés, de la manière que l'on exposera ci-dessous, par rapport à ces configurations connues.

Par ailleurs, le recueil est opéré dans des bandes de fréquences différentes : basse fréquence pour la mesure de MV, fréquence plus élevée pour la mesure d'impédance transseptale selon l'invention. Cette modification de la bande de fréquences peut résulter d'un déplacement des points de coupure du filtre, modification très aisée à réaliser par des moyens logiciels si le filtrage est un filtrage numérique.

Il est donc possible de réutiliser la chaîne de mesure de ventilation-minute classique préexistante du dispositif, en récupérant le signal de mesure avant l'étage de filtrage MV, permettant ainsi une mise en oeuvre de l'invention sans surcoût important.

Le courant injecté pour la mesure d'impédance selon l'invention est par exemple un courant de 40 µA délivré sous la forme d'une impulsion de largeur 5 µs.

La configuration de mesure (injection/recueil) selon l'invention est une configuration tripolaire, avec un point commun à l'injection et au recueil.

La configuration actuellement préférée, illustrée figure 1, est :
- injection entre VG- et AD-,
- recueil entre VG- et AD+.

En d'autres termes, le point commun, c'est-à-dire la référence de mesure, est l'électrode distale ventriculaire VG-.

Dans la variante de la figure 2, on a choisi comme point commun l'électrode proximale auriculaire AD+, c'est-à-dire que la configuration est :
- injection entre AD+ et VG-,
- recueil entre AD+ et VG+

Par ailleurs, pour toutes les configurations que l'on vient de décrire, il est possible d'inverser le rôle des électrodes proximales (+) et des électrodes distales (-).

Cette mesure d'impédance transseptale permet de disposer d'un signal corrélé aux variations du volume ventriculaire gauche, sans avoir besoin de mesurer les variations du volume ventriculaire droit.

Il est d'autant plus avantageux de disposer directement d'informations sur le comportement hémodynamique du ventricule gauche, qu'il s'agit du ventricule prédominant, qui supporte la pression artérielle (le ventricule droit ne reflète la pression artérielle que de façon indirecte et décalée temporellement par rapport au ventricule gauche).

Il est toutefois possible si on le souhaite d'inverser la configuration en opérant entre ventricule droit et oreillette gauche, pour obtenir ainsi une mesure du débit dans le coeur droit (qui est, en valeur moyenne, égal à celui du coeur gauche).

Le signal recueilli peut être utilisé pour piloter la fréquence de stimulation et/ou le délai atrioventriculaire et/ou le délai interventriculaire dans le cas d'une stimulation biventriculaire. L'ajustement de ces divers paramètres se fait bien entendu dans le sens procurant le débit cardiaque maximal.

Le dispositif peut en outre comprendre des moyens pour détecter des arythmies ventriculaires en donnant une information concernant l'hémodynamique ce qui permet d'opérer une discrimination entre, d'une part, un effort accompagné d'une fréquence de détection élevée et, d'autre part, un trouble du rythme accompagné d'une baisse du débit cardiaque.

## Revendications

1. Un dispositif médical implantable actif, notamment un stimulateur cardiaque, défibrillateur et/ou cardioverteur et/ou dispositif multisite dans lequel des électrodes sont placées en une pluralité de sites respectifs distincts comportant au moins un site ventriculaire gauche et un site auriculaire droit, ou bien au moins un site ventriculaire droit et un site auriculaire gauche, ces électrodes étant reliées à un circuit de recueil de signaux cardiaques, pour détecter un potentiel de dépolarisation, ainsi qu'à un circuit de stimulation, pour appliquer des impulsions de stimulation sur au moins certains desdits sites,
ce dispositif comportant en outre des moyens d'évaluation du débit cardiaque par mesure d'impédance transseptale entre ventricule gauche et oreillette droite, ou bien entre ventricule droit et oreillette gauche respectivement, ces moyens opérant par injection d'un courant (16) entre un site auriculaire et un site ventriculaire et recueil d'un potentiel différentiel (20) entre un site auriculaire et un site ventriculaire,
dispositif **caractérisé en ce que** les moyens de mesure d'impédance transseptale sont des moyens aptes à opérer ladite injection de courant entre:
- un site commun ventriculaire (VG- ; VG+) et un site propre auriculaire d'injection (AD- ; AD+), et à opérer ledit recueil du potentiel différentiel entre ledit site commun ventriculaire (VG- ; VG+) et un site propre auriculaire de recueil (AD+ ; AD-); ou
- un site commun auriculaire (AD+ ; AD-) et un site propre ventriculaire d'injection (VG-, VG+), et à opérer ledit recueil du potentiel différentiel entre ledit site commun auriculaire (AD+ ; AD-) et un site propre ventriculaire de recueil (VG+ ; VG-).

2. Le dispositif de la revendication 1, comprenant en outre des moyens pour faire varier la fréquence d'application des impulsions de stimulation, opérant en réponse à l'impédance transseptale mesurée dans le sens de l'amélioration du débit cardiaque.

3. Le dispositif de la revendication 1, comprenant en outre des moyens pour faire varier le délai atrioventriculaire d'application des impulsions de stimulation, opérant en réponse à l'impédance transseptale mesurée dans le sens de l'amélioration du débit cardiaque

4. Le dispositif de la revendication 1, comprenant en outre des moyens pour faire varier le délai interventriculaire d'application des impulsions de stimulation respectives des ventricules droit et gauche, opérant en réponse à l'impédance transseptale mesurée dans le sens de l'amélioration du débit cardiaque.

5. Le dispositif de la revendication 1, comprenant en outre des moyens de détection d'arythmies ventriculaires et de discrimination entre, d'une part, efforts accompagnés d'une fréquence cardiaque élevée et, d'autre part, troubles du rythme accompagnés d'une baisse du débit cardiaque détectée par les moyens de mesure d'impédance transseptale.

## Claims

1. An active implantable medical device, in particular a pacemaker, defibrillator and/or cardioverter and/or a multisite device, wherein electrodes are placed in a plurality of distinct respective sites comprising at least one left ventricular site and one right atrial site, or at least one right ventricular site and one left atrial site, said electrodes being connected to a cardiac signal collection circuit, to detect a potential of depolarization, and to a stimulation circuit, to apply stimulation pulses on at least some of said sites,
said device further comprising means for evaluating cardiac output by measuring a trans-septum impedance between the left ventricle and the right atrium, or the right ventricle and the left atrium, respectively, wherein said means operates by injecting a current (16) between an atrial site and a ventricular site and collecting a differential potential (20) between an atrial site and a ventricular site,
said device being **characterised in that** the means for measuring the trans-septum impedance is a means able to operate said current injection between:
- a common ventricular site (VG-; VG+) and a dedicated atrial injection site (AD-; AD+), and to operate said collection of the differential potential between said common ventricular site (VG-; VG+) and a dedicated atrial collection site (AD+; AD-); or
- a common atrial site (AD+; AD-) and a dedicated ventricular injection site (VG-, VG+), and to operate the collection of the differential potential between said common atrial site (AD+; AD-) and a dedicated ventricular collection site (VG+; VG-).

2. The device of claim 1, further comprising means for varying the application frequency of the stimulation pulses, operating in response to the measured trans-septum impedance in the direction of improvement of the cardiac output.

3. The device of claim 1, further comprising means for varying the atrioventricular delay of application of the stimulation pulses, operating in response to the measured trans-septum impedance in the direction of improvement of the cardiac output.

4. The device of claim 1, further comprising means for varying the interventricular delay of application of the respective stimulation pulses of the right and left ventricles, operating in response to the measured trans-septum impedance in the direction of improvement of the cardiac output.

5. The device of claim 1, further comprising means for detecting ventricular arrhythmia and for discriminating between, on the one hand, efforts accompanied by an increased heart rate and, on the other hand, rhythm disorders accompanied by a decrease of the cardiac output detected by the means for measuring trans-septum impedance.

## Patentansprüche

1. Aktive implantierbare medizinische Vorrichtung, insbesondere Herzschrittmacher, Defibrillator und/oder Kardioverter und/oder "Multisite"-Vorrichtung, in welcher Elektroden an einer Vielzahl von jeweiligen getrennten Sitzen angeordnet sind, die wenigstens einen Sitz in der linken Herzkammer und einen Sitz im rechten Herzvorhof oder wenigstens einen Sitz in der rechten Herzkammer und einen Sitz im linken Herzvorhof umfassen, wobei diese Elektroden mit einer Schaltung zur Erfassung von Herzsignalen verbunden sind, um ein Depolarisationspotential zu erfassen, sowie mit einer Stimulationsschaltung, um Stimulationsimpulse an wenigstens bestimmte der Sitze abzugeben,
wobei diese Vorrichtung außerdem Mittel zur Ermittlung des Herzdurchsatzes durch Messung der transscptalcn Impedanz zwischen jeweils der linken Herzkammer und dcm rechten Herzvorhof oder der rechten Herzkammer und dem linken Herzvorhof umfasst, wobei diese Mittel durch Einleitung eines Stromflusses (16) zwischen einem Herzvorhofsitz und einem Herzkammersitz und Erfassung eines Spannungsunterschieds (20) zwischen einem Herzvorhofsitz und einem Herzkammersitz wirken,
wobei die Vorrichtung **dadurch gekennzeichnet ist, dass** die Mittel zur Messung der transseptalen Impedanz Mittel sind, die geeignet sind, die Stromeinleitung durchzuführen zwischen
- einem gemeinsamen Herzkammersitz (VG-; VG+) und einem für die Einleitung eigenen Herzvorhofsitz (AD-; AD+), und die Erfassung des Spannungsunterschieds zwischen dem gemeinsamen Herzkammersitz (VG-; VG+) und einem für die Erfassung eigenen Herzvorhofsitz (AD+; AD-) vorzunehmen; oder
- einem gemeinsamen Herzvorhofsitz (AD+; AD-) und einem für die Einleitung eigenen Herzkammersitz (VG-; VG+), und die Erfassung des Spannungsunterschieds zwischen dem gemeinsamen Herzvorhofsitz (AD+; AD-) und einem für die Erfassung eigenen Herzkammersitz (VG+; VG-) durchzuführen.

2. Vorrichtung nach Anspruch 1, außerdem Mittel umfassend, um die Frequenz der Anwendung durch Stimulationsimpulse variieren zu lassen, die in Antwort auf die gemessene transseptale Impedanz in Richtung der Verbesserung des Herzdurchsatzes wirken.

3. Vorrichtung nach Anspruch 1, außerdem Mittel umfassend, um die atrioventrikuläre Verzögerung der Anwendung der Stimulationsimpulse variieren zu lassen, die in Antwort auf die gemessene transseptale Impedanz in Richtung der Verbesserung des Herzdurchsatzes wirken.

4. Vorrichtung nach Anspruch 1, außerdem Mittel umfassend, um die interventrikulüre Verzögerung der Anwendung der Impulse zur jeweiligen Stimulation der rechten und linken Herzkammer variieren zu lassen, die in Antwort auf die gemessene transseptale Impedanz in Richtung der Verbesserung des Herzdurchsatzes wirken.

5. Vorrichtung nach Anspruch 1, außerdem mit Mitteln zur Detektion von ventrikulären Arrhythmien und zur Unterscheidung zwischen einerseits von erhöhter Herzfrequenz begleiteten Anstrengungen und andererseits Rhythmusstörungen, die von einer Verringerung des Herzdurchsatzes bcgleitet sind, welche durch die Mittel zur Messung der transseptalen Impedanz detektiert wird.
